# EUROPEAN PATENT SPECIFICATION

(11) **EP 0 259 031 B1**
(45) Date of publication and mention of the grant of the patent: **09.11.1994**
(21) Application number: 87307095.7
(22) Date of filing: 11.08.1987
(51) Int. Cl.: C12Q 1/68, G01N 33/574, G01N 33/68, C07H 21/00

(54) **Human DNA in the diagnosis of retinoblastoma**
Menschliche DNS für die Diagnose von Glioma-retinae
ADM humain pour le diganostic du rétinoblastome

(30) Priority: 11.08.1986 US 895163
(43) Date of publication of application: 09.03.1988
(60) Divisional application: 94103625.3
(73) Proprietor: MASSACHUSETTS EYE & EAR INFIRMARY, Boston, MA 02114 (US); WHITEHEAD INSTITUTE FOR BIOMEDICAL RESEARCH, Cambridge, MA 02142 (US)
(72) Inventor: Dryja, Thaddeus P., Milton, MA 02186 (US); Friend, Stephen, Sommerville, MA 02143 (US)
(74) Representative: Sheard, Andrew Gregory

(56) References cited:
- EP-A- 0 293 266
- RECESSIVE ONCOGENES AND TUMOUR SUPRESSION, Cold Spring Harbour Laboratory Press, 1989; pp. 117-123#
- ONCOGENES, Jones & Bartlett Publishers Inc., 1990; pp. 121-139#
- SCIENCE, vol. 241, 1988; pp. 293-294#
- SCIENCE, vol. 241, 1988; pp. 218-221#
- ONCOGENE, vol. 3, 1988; pp. 471-475#
- SCIENCE, vol. 243, 1989; pp. 937-940#
- PROCEEDINGS OF THE NATL. ACADEMY OF SCIENCES USA, vol. 87, 1990; pp. 2775-2779#
- PROCEEDINGS OF THE NATL. ACADEMY OF SCIENCES USA, vol. 88, 1991; pp. 5257-5261#
- SCIENCE, 13 March 1987; p. 1323#
- CELL, vol. 54, 1988; pp. 275-283#
- NATURE, vol. 329, 1987; pp. 642-645#
- NATURE, vol. 334, 1988; pp. 124-129#
- SCIENCE, vol. 243, 1989; pp. 934-937#
- SYMPOSIA ON FUNDAMENTAL CANCER RESEARCH, vol. 39, 1986, New York, NY (US); T.P. DRYJA et al., pp. 115-119#
- NATURE, vol. 323, 16 October 1986, London (GB); S.F. FRIEND et al., pp. 643-646#
- SCIENCE, vol. 236, 26 July 1987, Lancaster, PA (US); Y.T. FUNG et al., pp. 1657-1661#
- SCIENCE, vol. 235, no. 4794, 13 March 1987, Lancaster, PA (US); W.H. LEE et al., pp. 1394-1399#

## Description

This invention relates to methods of detection and treatment of a defective human gene related to cancer, in particular, retinoblastoma.

Nature 305 (1983) 779-84 discloses that a mutation at the retinoblastoma locus (Rb-l), mapped to human chromosome 13 band ql4, results in a predisposition to retinoblastoma.

Retinoblastoma is a neoplastic condition of the retinal cells, observed almost exclusively in children between the ages of 0 and 4 years. If untreated, the malignant neoplastic retinal cells in the intraocular tumor travel to other parts of the body, forming foci of uncontrolled growth which are always fatal. The current treatment for a retinoblastoma is enucleation of the affected eye if the intraocular tumor is large; for small intraocular tumors, radiation therapy, laser therapy, or cryotherapy is preferred. There is no known successful treatment for metastatic retinoblastoma. Hence, early diagnosis of retinoblastoma to allow treatment before the tumor spreads outside the eye is crucial.

There is evidence that retinoblastoma is caused by the functional loss of both homologous copies of the retinoblastoma (Rb) gene. Thus, individuals carrying one defective allele of the Rb gene are genetically predisposed to the disease. Children who have had one eye affected by retinoblastoma or who are related to someone with retinoblastoma may be genetically predisposed and therefore at risk of developing the disease. These individuals routinely are tested for retinoblastoma every 2-3 months by an ocular examination procedure which requires placing the child under general anesthesia.

The invention relates to the use of genetic material corresponding to a normal human retinoblastoma gene or a unique subregion thereof in the preparation of material for use in a method of screening human patients comprising comparing the DNA of said patients with the said gene or subregion. Also encompassed are vectors comprising genetic material corresponding to a normal retinoblastoma gene, or a unique subregion thereof.

In general, the invention features a method of screening human patients by comparing the DNA of these patients with the isolated normal human retinoblastoma (Rb) gene or a unique subregion thereof (the term "unique subregion" means a DNA sequence found in the Rb gene and not elsewhere in the human genome). This comparison allows detection of defective Rb alleles in the patients, to determine whether these patients need continual monitoring by the conventional examination procedure. More importantly, this comparison will identify those patients who do not have a defective Rb allele and thus are not at risk of developing retinoblastoma and do not have to be examined by the conventional procedure.

Preferably, the comparison between the patient's DNA and the normal Rb gene involves testing the patient's DNA with the isolated Rb gene to detect either large deletions or, alternatively, small deletions or point mutations in the Rb locus. To test for large deletions in a patient's Rb allele, the patient's DNA preferably is analyzed by DNA hybridization using probes made from the isolated normal Rb gene. According to the invention, small deletions or point mutations preferably are detected by either of two techniques. The nucleotide sequences of the patients' Rb alleles and the normal Rb gene can be determined and compared for differences. Alternatively, the patient's DNA is probed with the normal Rb gene and any mismatches in the resulting heteroduplexes are identified.

Also, the isolated normal human retinoblastoma gene can be used to produce the normal Rb gene product for protein therapy of individuals determined to have a defective Rb allele.

In another aspect, the invention features a method of detecting the presence, in a tumor sample, of a retinoblastoma protein the absence of which is associated with a distinct set of neoplasms. The method comprises producing an antibody to the Rb protein, contacting the antibody with the tumor sample, and detecting immune complexes as an indication of the presence of the protein in the tumor sample. If a tumor lacks the Rb gene product, no immune complexes will be found, and one may conclude that the tumor was the result of mutant Rb alleles. This limits the pathologic diagnosis to those tumors known to be caused by mutant Rb alleles, such as retinoblastoma, osteosarcoma, and some undifferentiated tumors of unknown cellular origin. A more exact categorization of pathologic diagnosis of human tumors will result.

### Description of the Preferred Embodiments

The drawings first will be briefly described.

### Drawings

Fig. 1 is a pictorial representation of the autoradiogram from a Northern blot probed with p7H30.7R;
Fig. 2 is a diagrammatic representation of the restriction map of the insert in the clone p4.7R;
Fig 3 is a pictorial representation of the autoradiogram from a Northern blot probed with p4.7R;
Fig. 4 is a diagrammatic representation of the vectors p2AR3.8 and p2AR0.9 of the invention;
Fig. 5 is a diagrammatic representation of the mismatch detection technique;
Fig. 6 is a diagrammatic representation of an example denaturing gel used in mismatch detection.
Fig. 7 is the sequence of the normal Rb gene, with flanking regions.

### Isolation of the Normal Rb Gene

The genetic locus involved in causing retinoblastoma has been assigned to the q14 band of human chromosome 13 (Sparkes et al., Science 208:1042 (1980). A cDNA clone, p4.7R, from this region of DNA has been shown to carry Rb gene sequences. This clone was obtained by the following general techniques.

### Isolation of cDNA Clone p4.7R

The human DNA probe pH3-8, isolated from a human chromosome 13 lambda phage library (Lalande et al., 1984, Cancer Genet. Cytogenet. 13:283), was used in a chromosome walking technique to isolate and map 30 kb of genomic DNA surrounding the H3-8 sequence. One fragment generated by this technique, named p7H30.7R, was found to recognize a DNA sequence in the mouse genome as well as within human chromosome 13 (Dryja et al., 1986, Proc. Nat. Acad. Sci. USA in press). The homology of p7H30.7R to both human and mouse DNA suggested that p7H30.7R contained coding sequences of a structural gene.

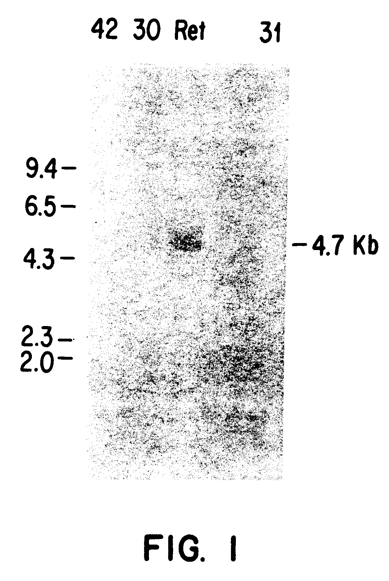

To test this possibility, p7H30.7R was radiolabeled and used to probe a Northern blot of RNA isolated from three retinoblastoma tumors (#42, #30, and #31) and an adenovirus 12-transformed human embryonic retinal cell line (Ret) (Vaessen et al., 1986, EMBO Journal 5:335). The p7H30.7R probe hybridized to an RNA transcript of approximately 4.7 kb from the retinal cell line, but did not hybridize to any RNA transcripts from the three tumor samples (Fig. 1).

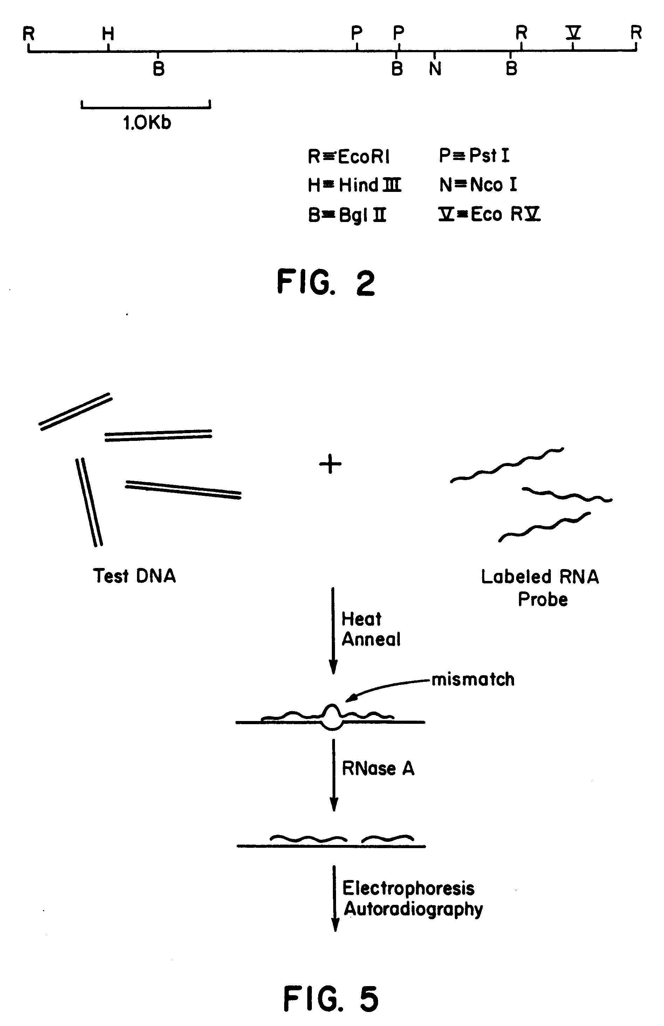

Subsequently, RNA isolated from the adenovirus-transformed retinal cell line was used to construct a cDNA library. This library was screened with the labeled p7H30.7R probe. Several cDNA clones were isolated which had similar restriction maps. The longest of these, p4.7R, contained 4.7 kb of genomic DNA. The physical map of p4.7R is shown in Fig. 2.

### Characterization of p4.7R

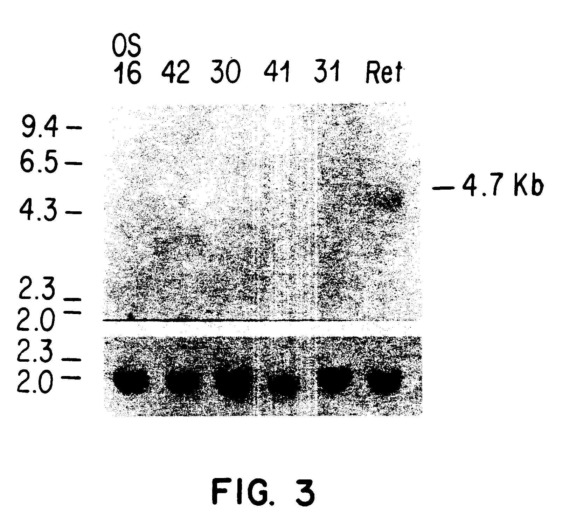

The p4.7R clone was used to screen RNA transcripts isolated from retinoblastomas (#42, #30, #41, #31), an osteosarcoma (#16), and the adenovirus-transformed retinal cells (Ret). As shown in Fig. 3, the p4.7R probe detected, in a Northern blot analysis of isolated RNA's, a transcript in the transformed retinal cells which is not present in the four retinoblastoma and one osteosarcoma cell samples. The bands at ∼ 2.0 kb were detected by reprobing the Northern blot, after washing, with a probe that detects rat tubulin (to demonstrate the presence of RNA in the blot).

The p4.7R clone also was used to screen genomic DNA. DNA was isolated from a set of tumors from 50 unrelated individuals, consisting of 40 retinoblastomas, 8 osteosarcomas, and 2 undifferentiated tumors of unknown cellular origin arising in patients with hereditary retinoblastoma. The isolated samples of DNA were digested with HindIII and analyzed by Southern blot hybridization using radiolabeled p4.7R as the probe. This analysis revealed three types of deviant patterns of the genomic DNA restriction fragments: totally absent fragments, representing apparent homozygous deletions; under-represented fragments, representing apparent heterozygous deletions; and fragments of altered size, reflecting either partial deletion or an alteration of a restriction site. At least 30% of the tumor DNA's exhibited one of these abnormalities. In comparison, Southern blot analysis of leukocyte DNA from 18 normal individuals showed a uniform pattern of restriction fragments.

The above results indicate that p4.7R detects the Rb gene. The deletion pattern in one osteosarcoma DNA sample provided particularly good evidence that p4.7R detects the Rb gene. This DNA sample is homozygous for a deletion that maps entirely within the p4.7R region. It is highly unlikely that the osteosarcoma phenotype arose due to a mutation independent of this deletion. Since the deletion is limited to the p4.7R region, this region must contain the Rb gene which, when mutated, produces non-functional Rb-encoded protein. The absence of functional Rb protein allows the neoplastic phenotype to develop.

### Use

The p4.7R sequences can be used, according to the invention, to screen individuals for the presence of a mutated
allele of the Rb gene. This screening procedure will allow individuals having a risk of developing retinoblastoma--because of family history or a previous incidence of retinoblastoma in one eye--to determine the need for routine testing by the current ocular examination procedure. Only if the screening procedure determines that the individual possesses a mutant Rb allele will the examination procedure need to be conducted on a regular basis. Those with two normal Rb alleles can discontinue examination, as the risk of developing retinoblastoma in an individual with two normal copies of the Rb gene is approximately 1 in 20,000, or 0.005%, compared to a risk of 80%-90% if an individual has an Rb allele containing a mutation sufficient to inactivate the allele. Thus, a substantial percentage of individuals who are currently examined regularly are not actually at a greater risk than the general population: neither a family history of nor a previous incidence of retinoblastoma is conclusive evidence that an individual has the genetic predisposition to the disease. Therefore, such individuals, actually carrying two normal copies of the Rb gene, have been repeatedly undergoing the expensive and traumatic ocular examination procedure needlessly.

The screening procedure according to the invention preferably can be of two major types: (1) testing an individual's DNA for deletions in the Rb locus large enough to interfere with hybridization to an Rb probe, and (2) testing an individual's DNA for small deletions or point mutations in the Rb locus.

In addition to screening, the invention has the potential to provide protein therapy for those individuals determined to contain a mutant Rb allele and who therefore are at risk of developing retinoblastoma.

An additional use of the invention, as mentioned above, is in immunodiagnosis to determine, for example, whether a certain tumor is the result of an Rb gene abnormality. Since osteosarcomas and certain undifferentiated tumors can result from detectable lesions in the Rb gene, the immunodiagnosis can be used to aid in the diagnosis of such tumors.

Illustrative examples are given below.

### Example 1: Southern Blot Analysis

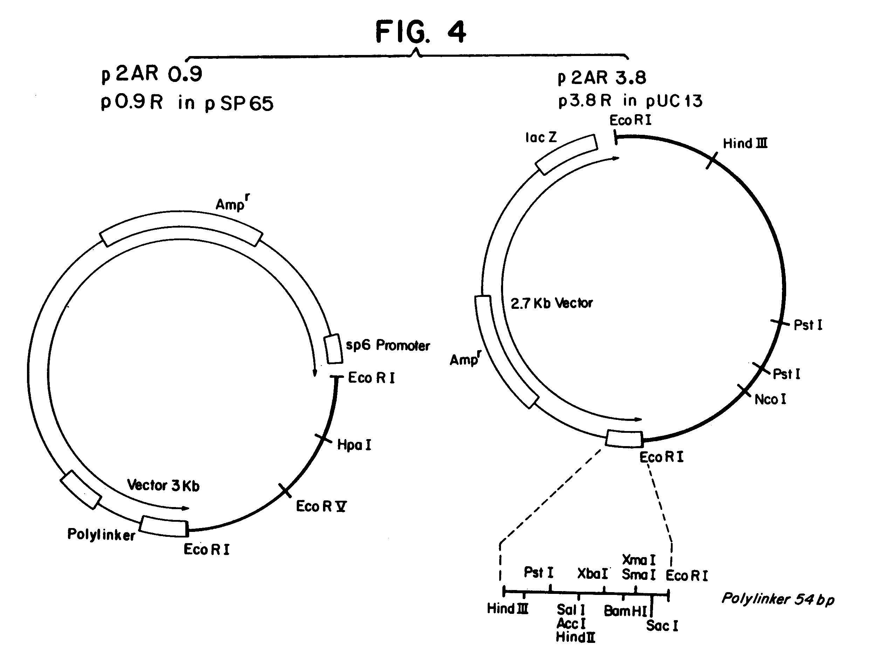

To detect large deletions in the Rb locus, a Southern Blot analysis is carried out on DNA obtained from an individual to be tested. The DNA is obtained from peripheral leucocytes or, if the patient has had a tumor in one eye, from the tumor. To examine leucocyte DNA, a 10 ml blood sample is obtained from the individual, and the genomic DNA is isolated from the leucocytes in the sample, according to standard techniques. This DNA is digested with a restriction endonuclease, e.g., HindIII, run on an agarose electrophoresis gel, and transferred to a nitrocellulose filter by blotting. The DNA on the filter is then probed with radiolabeled p2AR3.8 and, separately, p2AR0.9, containing subfragments from p4.7R obtained by EcoRl digestion (Fig. 4); it is preferred to use two or more subfragments separately rather the entire p4.7R insert, in order to better define the location of any abnormalities detected. Autoradiograms of the probed filter give a restriction map of the Rb locus in the somatic or tumor DNA of the tested individual.

This restriction map then is compared with a control restriction map, determined by using the same restriction enzyme digestion and probe. A suitable control can be DNA obtained from the adenovirus-transformed retinal cell line or leucocyte DNA from a set of normal individuals. If the tested individual has an Rb allele containing a significantly large deletion, the above restriction map of his DNA, compared with the control, will contain an additional band or bands, and/or a band or bands that have lost 50% of their intensity, caused by a change in the size, or total elimination, of one or more restriction fragments by the deletion in one allele at the Rb locus.

Thus, this screening procedure by Southern analysis will detect the existence of non-functional Rb alleles which have large deletions. If this analysis indicates that the tested DNA from an individual has a restriction map different from the control map, there is a great probability that the individual contains a non-functional, mutant Rb allele. The individual must be monitored closely thereafter for the development of retinoblastoma.

If the test restriction map appears identical to the control, a different screening procedure can be performed on the individual's DNA to determine if the individual contains an Rb allele having a small deletion or point mutation, which is sufficient to inactivate the allele but not to prevent hybridization with a probe. This screening procedure is described in the following example.

### Example 2: Rb Locus Fine Structural Analysis

To examine an individual's DNA for small deletions or point mutations in the Rb locus, both homologs of the Rb gene from the individual preferably are cloned. The cloned alleles then can be tested for the presence of sequence differences from the normal allele, represented by p4.7R, by one of the following two methods: (1) the nucleotide sequence of both the cloned alleles and p4.7R are determined and then compared, or (2) RNA transcripts from p4.7R are hybridized to single stranded whole genomic DNA from an individual to be tested, and the resulting heteroduplex is treated with RNase A and run on a denaturing gel to detect the location of any mismatches. In more detail, these methods are carried out as follows:

### (1) Cloning Rb alleles

The alleles of the Rb gene in an individual to be tested are cloned using conventional techniques. A common method, for example, employs the bacteriophage vector EMBL3 (Frischauf et al., 1983, J. Mol. Biol. 170:827). A 10 ml blood sample is obtained from the individual, and the genomic DNA is isolated from the cells in this sample. This DNA is partially digested with MboI to an average fragment size of approximately 20 kb. Fragments in the range from 18-21 kb are isolated. The resulting MboI-ended fragments are ligated into the EMBL3 vector DNA which has been completely digested with BamHI, treated with alkaline phosphatase, and heated to 68°C for 10 minutes to disrupt the cohesive ends. This ligation mix is used in an in vitro lambda packaging reaction, and the packaged phage are amplified by growing a plate stock. [This cloning technique is described generally in Maniatis et al., Molecular Cloning: A Laboratory Manual, Cold Spring Harbor Publications, pp 256-293 (1982).]

Approximately 5 x 10⁵ pfu from this plate stock are used to infect 3 ml of E. coli cells at ∼ 1.5 x 10⁹ cells/ml in 0.01M MgSO₄, and the infection mix is incubated at 37°C for 20 minutes. 65 ml melted top agar at 47°C is added, and the mixture is plated onto ten 150 mm plates containing freshly poured and dry bottom agar. The agar plates are incubated until the plaques reach a diameter of ∼ 1.5 mm and are just beginning to contact one another (approximately 10-12 hours).

Duplicate circular nitrocellulose filters (Millipore™ HAWP) are placed gently on the surface of each agar plate to bind the bacteriophage DNA. The filters are carefully removed after 1 minute, placed into a denaturing solution (1.5m NaCl, 0.5M NaOH) for 30 seconds, neutralized for 5 minutes (1.5M NaCl, 0.5M Tris-Cl pH 8.0), and dried under vacuum at 80°C for 2 hours.

These nitrocellulose filters then are probed with radiolabeled p4.7R by hybridization and autoradiography. Plaques which show hybridization to the p4.7R probe are plaque-purified and rescreened according to the above procedure. Positive plaques from the rescreening are isolated and used to prepare DNA putatively containing Rb alleles from the individual.

The MboI genomic inserts in these isolated EMBL3 vector DNA samples are tested for the location of the sequences homologous to p4.7R by Southern analysis. DNA samples containing the entire Rb gene region are selected, and the appropriate restriction fragments containing the Rb gene from these samples are subcloned into a suitable vector, such as pUC9. These subclones thus contain copies of one or both Rb alleles from the DNA of the individual to be tested. To determine if both alleles are represented, the initial phage isolates are tested for the existence of restriction polymorphism. These subcloned alleles are then examined for differences from p4.7R by one of the following techniques.

### (2) Sequence Comparison

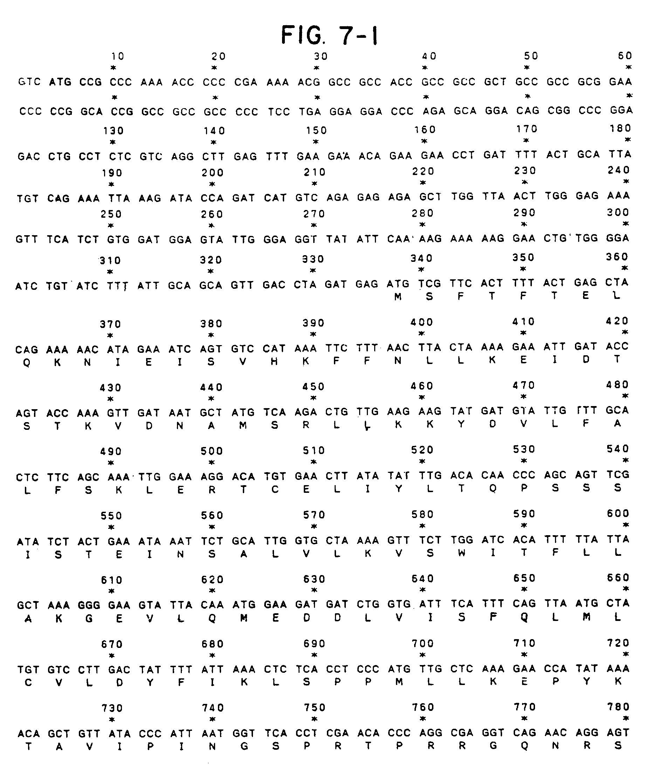

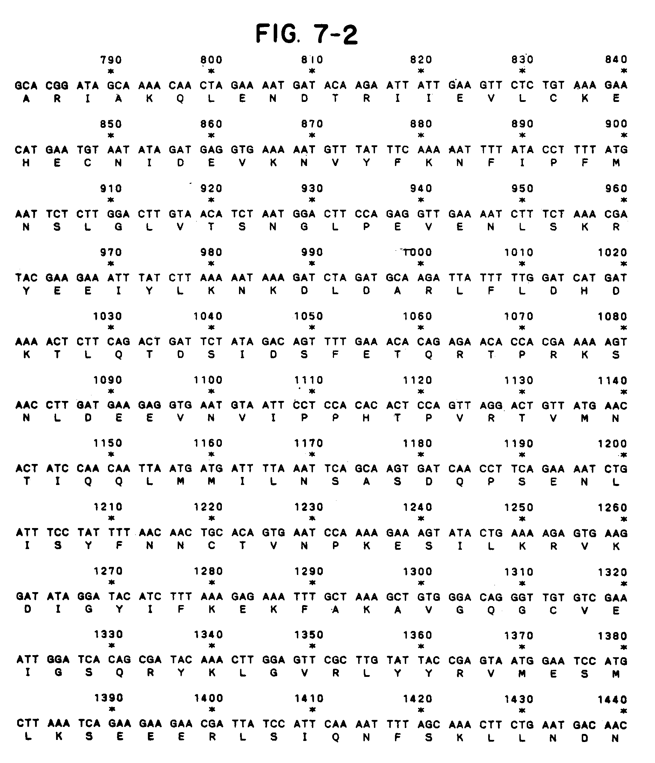

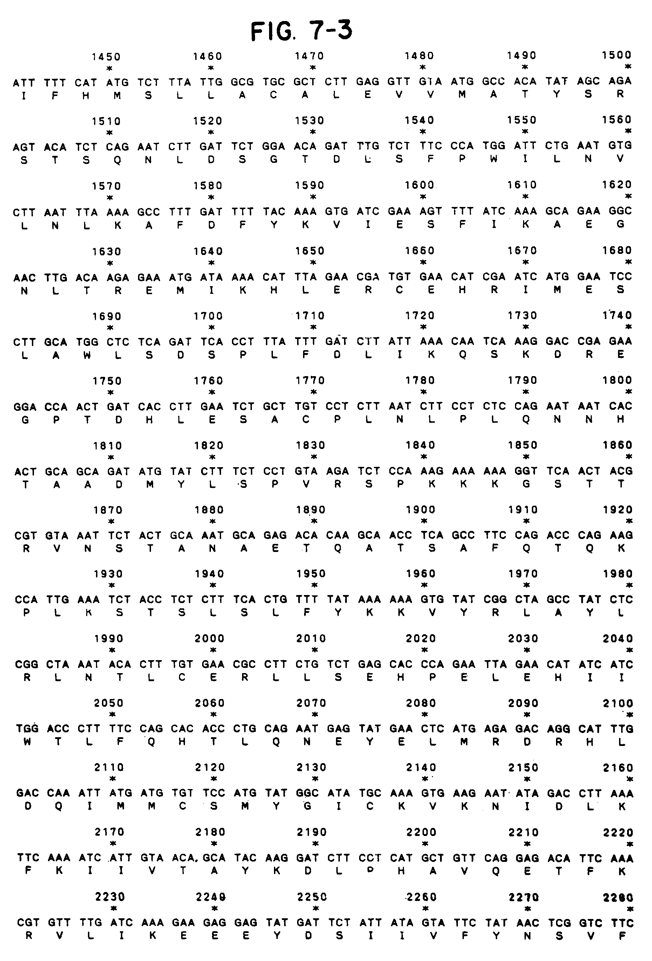

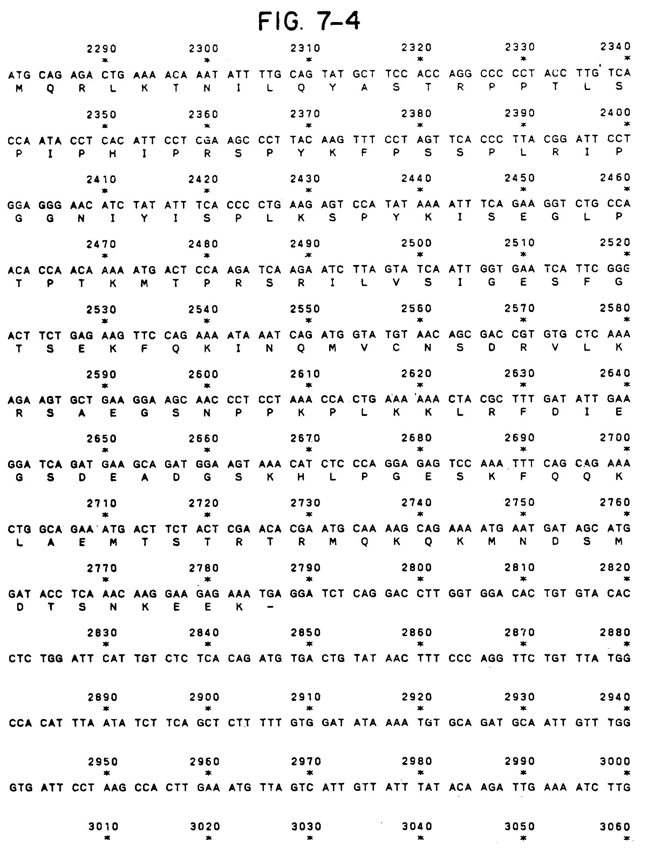

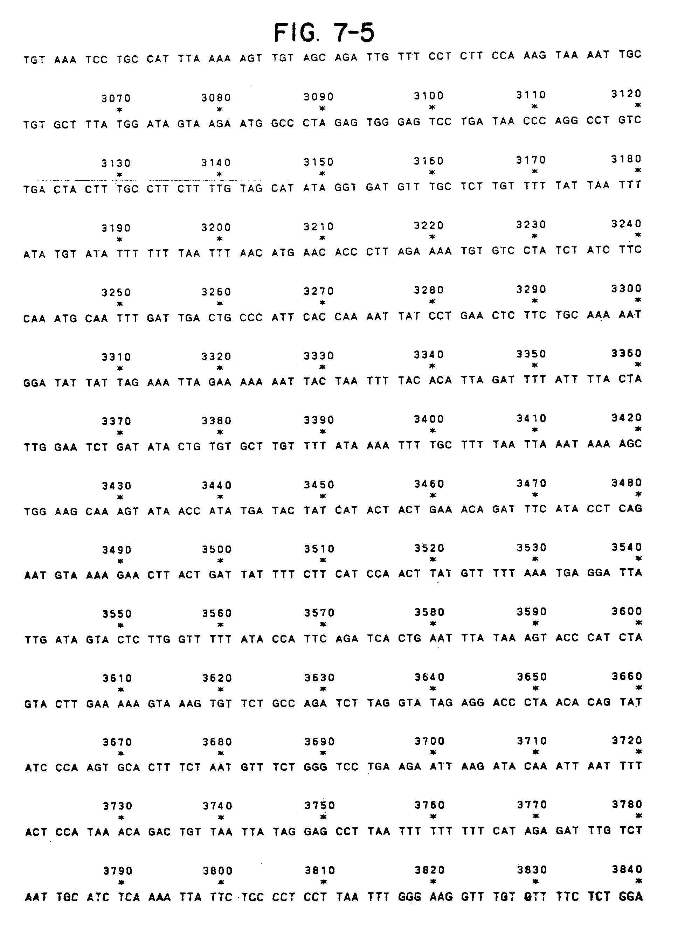

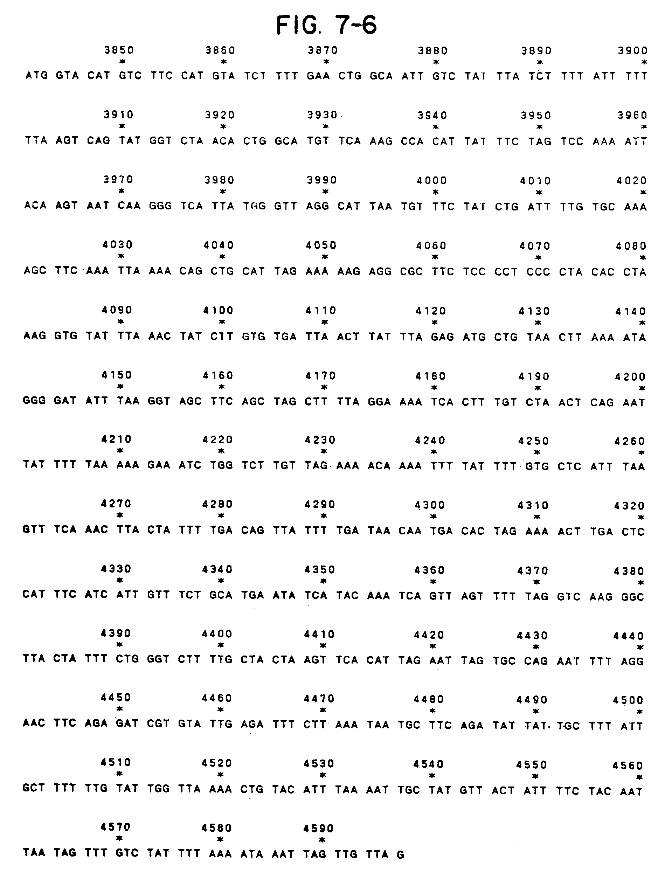

First, the nucleotide sequence of the normal Rb gene in p4.7R is determined by subcloning restriction fragments of ∼ 500 bp from p4.7R into an M13mp8 phage vector and sequencing these subclones by the dideoxy technique (Sanger et al., 1977, Proc. Nat. Acad. Sci USA 74:5463). A composite sequence of the Rb gene then can be assembled from these individual subclone sequences. This sequence is given in Fig. 7 which also shows flanking regions.

The isolated Rb gene alleles are sequenced according to the following procedure. Restriction fragments (∼ 2kb) of the allele are subcloned into the M13mp8 vector, and short stretches (∼500 bp) are sequenced individually using small restriction fragments isolated from p4.7R as the primers in the dideoxy sequencing reactions. The composite nucleotide sequence of the isolated allele then can be constructed from these individually-primed sequences. This sequence is compared directly with the sequence of the normal Rb gene, determined from p4.7R, to determine if any deletions or point mutations exist in the isolated allele.

### (3) Ribonuclease Cleavage of Mismatches

An alternative method of comparing the allelic DNA with the normal Rb gene employs RNase A to detect the existence of differences between the p4.7R sequence and the allele sequence. This comparison is performed in steps with small (∼ 500 bp) restriction fragments of p4.7R as the probe. First, p4.7R is digested with a restriction enzyme(s) that cuts the Rb gene sequence into fragments of approximately 500bp. These fragements are isolated on an electrophoresis gel and cloned individually, in both orientations, into an SP6 vector, such as pSP64 or pSP65 (Melton et al., 1984, Nucleic Acids Res. 12:7035). The SP6-based plasmids containing inserts of p4.7R fragments are transcribed in vitro using the SP6 transcription system, well known in the art, in the presence of [α-³²P]GTP, generating radiolabeled RNA transcripts of both strands of the cDNA of the Rb gene.

Individually, these RNA transcripts are used to form heteroduplexes with the allelic DNA, as follows. 50 ng of the allele subclone is digested with a restriction enzyme that cuts outside of the region covered by the RNA transcript probe to be used. This digested DNA is mixed with the radiolabeled RNA probe in 30 µl of hybrization buffer (80% formamide, 40 mM Pipes pH6.4, 0.4M NaCl, and 1mM EDTA) and the mixture is treated at 90°C for 10 minutes to denature the DNA. The mixture then is cooled slowly to 45°C and the RNA is allowed to anneal to the single-stranded DNA at 45°C for half an hour.

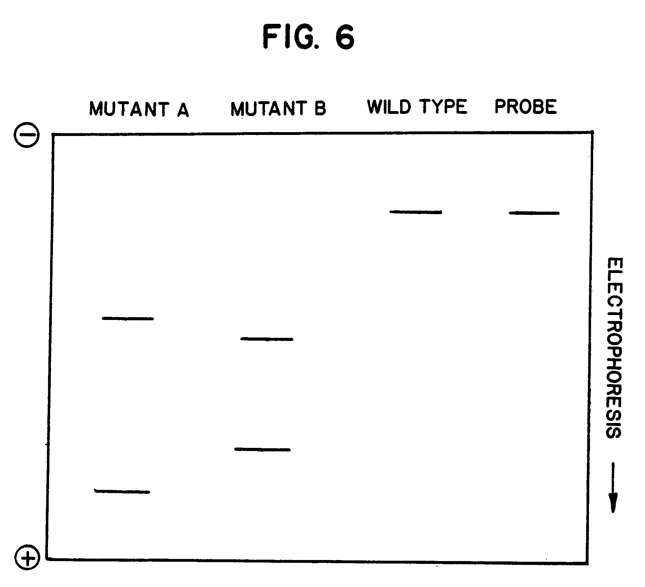

The RNA:DNA heteroduplexes next are treated with 350 µl of an RNase A solution (Sigma) (40 µg/ml in 10mM Tris-HCl pH7.5, 1mM EDTA, 0.2M NaCl, and 0.1M LiCl). The mixture is vortexed and incubated at 25°C for 30 minutes. The RNase A reaction is stopped by adding 10µl of proteinase K (10mg/ml) (Boehringer Mannheim) followed by incubation at 37°C for 20 minutes. Extraction with phenol-chloroform and ethanol precipitation of the aqueous layer yields a nucleic acid sample free from protein contamination. The precipitated sample is resuspended in 5µl and analyzed by denaturing polyacrylamide gel electrophoresis (4% polyacrylamide, 7M urea) (Fig. 5).

Mismatches that occur in the RNA:DNA heteroduplex, due to sequence differences between the p4.7R fragment and the Rb allele subclone from the individual, result in cleavage in the RNA strand by the RNase A treatment. Such mismatches can be the result of point mutations or small deletions in the individual's Rb allele. Cleavage of the RNA strand yields two or more small RNA fragments, which run faster on the denaturing gel than the RNA probe itself, as shown in Fig. 6.

In the above RNAse A technique, radiolabeled Rb gene RNA is hybridized to single strands of an individual's Rb allele which has been cloned into a vector. The RNase A technique is advantageous, however, because it also can be used without having to clone the Rb alleles. Preferably, genomic DNA is isolated from blood cells of the individual to be tested, and this genomic DNA is hybridized directly with the radiolabeled Rb RNA probes to determine sequence differences from the normal Rb gene, as follows. 5 µg of isolated, total genomic DNA is resuspended with the labeled RNA probe in 30 µl of hybridization buffer (80% formamide, 40mM Pipes pH6.4, 0.HM NaCl, and 1mM EDTA), and this hybridization mix is treated at 90°C for 10 minutes to denature the DNA. The mixture then is cooled slowly to 45°C and incubated at this temperature for 10 hours to allow hybridization of the RNA probe to the single-stranded DNA copies of the Rb allele. After hybridization, the RNase A treatment and electophoresis are performed as above. Mismatches in the heteroduplexes between the RNA probe and the genomic copies of the individual's Rb alleles are readily detected.

### Example 3: Production of Rb protein

Another use for the cloned cDNA of the normal Rb gene, as represented by p4.7R, is to produce the Rb protein. The Rb protein is produced by cloning the Rb cDNA from p4.7R into an appropriate mammalian expression vector, expressing the Rb protein from this vector in an in vivo expression system, and isolating the Rb protein from the medium or cells of the expression system.
General in vitro expression vectors and systems are well known in the art.

### Example 4: Immunodiagnosis

The Rb protein, produced as described above, is injected into a rabbit to produce anti-Rb antibody, which then is labeled, e.g., radioactively, fluorescently, or with an enzyme such as alkaline phosphatase. The labeled antibody is used to determine whether human tumors are of defective Rb gene origin. This can be carried out using any conventional technique. For example, the tumor sample can be liquified and tested against the labeled antibody using a conventional ELISA format. Alternatively, a tumor section can be fixed and reacted with labeled antibody, and any immune complexes then can be detected by autoradiography or fluorescence microscopy, depending on the type of label on the antibody. Tumors lacking an antigen reactive with the antibody to the Rb gene product are due to mutations of the retinoblastoma gene. Since the tumors known to be caused by a mutant Rb gene are few (including retinoblastoma and osteosarcoma), the differential diagnosis of tumors deficient in the Rb gene product is greatly limited by such a test.

### Deposits

The plasmids p2AR3.8 and p2AR0.9 were deposited on July 17, 1987 with the American Type Culture Collection, Rockville, Maryland, and assigned ATCC accession numbers 40,241 and 40,242, respectively.

Other embodiments are within the following claims.

## Claims (Claims for the following Contracting State(s): BE, CH, DE, FR, GB, GR, IT, LI, LU, NL, SE)

1. The use of genetic material corresponding to a normal human retinoblastoma gene or a unique subregion thereof in the preparation of material for use in a method of screening human patients for defective retinoblastoma alleles which method comprises comparing the DNA of patients with the gene or subregion.

2. The use claimed in claim 1 wherein the comparison is carried out by DNA hybridization.

3. The use claimed in claim 1 wherein the comparison is carried out by sequence comparison or sequence analysis.

4. The use claimed in claim 1 wherein the comparision is carried out by detection of mismatches in a heteroduplex between the patient's DNA and the normal gene or RNA transcripts from the normal gene.

5. A vector comprising genetic material corresponding to a normal human retinoblastoma gene, or a unique subregion thereof.

6. A vector as claimed in claim 5, wherein the subregion is at least 20 bp in length.

7. A method of detecting the presence, in a tumour sample, of a retinoblastoma protein the absence of which is associated with a neoplasm, the method comprising producing an antibody to the retinoblastoma protein, contacting the antibody with the tumour sample, and detecting immune complexes as an indication of the presence in the tumour sample of the protein.

8. A method of screening a human patient for the presence of a defective retinoblastoma (Rb) gene, the method comprising comparing the DNA of a patient with a normal human retinoblastoma gene or a unique subregion thereof.

9. A method according to claim 8 wherein the comparison is carried out by DNA hybridisation or by sequence comparison or analysis.

10. A method according to claim 8 wherein the comparison is carried out by detection of mismatches in a heteroduplex between the patient's DNA and the normal gene or RNA transcripts from the normal gene.

## Claims (Claims for the following Contracting State(s): AT, ES)

1. The use of genetic material corresponding to a normal human retinoblastoma gene or a unique subregion thereof in the preparation of material for use in a method of screening human patients for defective retinoblastoma alleles which method comprises comparing the DNA of patients with the gene or subregion.

2. The use claimed in claim 1 wherein the comparison is carried out by DNA hybridization.

3. The use claimed in claim 1 wherein the comparison is carried out by sequence comparison or sequence analysis.

4. The use claimed in claim 1 wherein the comparision is carried out by detection of mismatches in a heteroduplex between the patient's DNA and the normal gene or RNA transcripts from the normal gene.

5. A process for the preparation of a vector comprising genetic material corresponding to a normal human retinoblastoma gene, or a unique subunit thereof, the process comprising either linking successive nucleotides together and/or ligating poly- and/or oligo- nucleotides.

6. A process as claimed in claim 6, wherein the subregion is at least 20 bp in length.

7. A method of detecting the presence, in a tumour sample, of a retinoblastoma protein the absence of which is associated with a neoplasm, the method comprising producing an antibody to the retinoblastoma protein, contacting the antibody with the tumour sample, and detecting immune complexes as an indication of the presence in the tumour sample of the protein.

8. A method of screening a human patient for the presence of a defective retinoblastoma (Rb) gene, the method comprising comparing the DNA of a patient with a normal human retinoblastoma gene or a unique subregion thereof.

9. A method according to claim 8 wherein the comparison is carried out by DNA hybridisation or by sequence comparison or analysis.

10. A method according to claim 8 wherein the comparison is carried out by detection of mismatches in a heteroduplex between the patient's DNA and the normal gene or RNA transcripts from the normal gene.

## Patentansprüche (Patentansprüche für folgende(n) Vertragsstaat(en): BE, CH, DE, FR, GB, GR, IT, LI, LU, NL, SE)

1. Verwendung von genetischem Material, das einem normalen menschlichen Retinoblastomgen oder einem eindeutigen Teilbereich davon entspricht, zur Herstellung von Material zur Verwendung in einem Verfahren zum Überprüfen menschlicher Patienten auf defekte Retinoblastomallele, wobei das Verfahren den Vergleich der Patienten-DNA mit dem Gen oder Teilbereich umfaßt.

2. Verwendung nach Anspruch 1, wobei der Vergleich durch DNA-Hybridisierung durchgeführt wird.

3. Verwendung nach Anspruch 1, wobei der Vergleich durch Sequenzvergleich oder Sequenzanalyse durchgeführt wird.

4. Verwendung nach Anspruch 1, wobei der Vergleich durch den Nachweis von Fehlpaarungen in einem Heteroduplex zwischen der Patienten-DNA und dem normalen Gen oder RNA-Transkripten von dem normalen Gen durchgeführt wird.

5. Vektor umfassend genetisches Material, das einem normalen menschlichen Retinoblastomgen oder einem eindeutigen Teilbereich davon entspricht.

6. Vektor nach Anspruch 5, wobei der Teilbereich mindestens 20 bp lang ist.

7. Verfahren zum Nachweis eines Retinoblastomproteins in einer Tumorprobe, wobei die Abwesenheit des Proteins mit einem Neoplasma einhergeht und das Verfahren die Herstellung eines Antikörpers gegen das Retinoblastomprotein, das Inkontaktbringen des Antikörpers mit der Tumorprobe und den Nachweis von Immunkomplexen als Anzeichen des Vorhandenseins des Proteins in der Tumorprobe umfaßt.

8. Verfahren zum Überprüfen eines menschlichen Patienten auf das Vorhandensein eines defekten Retinoblastom (Rb)-gens, das den Vergleich der Patienten-DNA mit einem normalen menschlichen Retinoblastomgen oder einem eindeutigen Teilbereich davon umfaßt.

9. Verfahren nach Anspruch 8, wobei der Vergleich durch DNA-Hybridisierung oder durch Sequenzvergleich oder -analyse durchgeführt wird.

10. Verfahren nach Anspruch 8, wobei der Vergleich durch den Nachweis von Fehlpaarungen in einem Heteroduplex zwischen der Patienten-DNA und dem normalen Gen oder RNA-Transkripten von dem normalen Gen durchgeführt wird.

## Patentansprüche (Patentansprüche für folgende(n) Vertragsstaat(en): AT, ES)

1. Verwendung von genetischem Material, das einem normalen menschlichen Retinoblastomgen oder einem eindeutigen Teilbereich davon entspricht, zur Herstellung von Material zur Verwendung in einem Verfahren zum Überprüfen menschlicher Patienten auf defekte Retinoblastomallele, wobei das Verfahren den Vergleich der Patienten-DNA mit dem Gen oder Teilbereich umfaßt.

2. Verwendung nach Anspruch 1, wobei der Vergleich durch DNA-Hybridisierung durchgeführt wird.

3. Verwendung nach Anspruch 1, wobei der Vergleich durch Sequenzvergleich oder Sequenzanalyse durchgeführt wird.

4. Verwendung nach Anspruch 1, wobei der Vergleich durch den Nachweis von Fehlpaarungen in einem Heteroduplex zwischen der Patienten-DNA und dem normalen Gen oder RNA-Transkripten von dem normalen Gen durchgeführt wird.

5. Verfahren zur Herstellung eines Vektors, der genetisches Material umfaßt, das einem normalen menschlichen Retinoblastomgen oder einem eindeutigen Teilbereich davon entspricht, das entweder die Verknüpfung von aufeinanderfolgenden Nucleotiden und/oder die Ligierung von Poly- und/oder Oligonucleotiden umfaßt.

6. Verfahren nach Anspruch 5, wobei der Teilbereich mindestens 20 bp lang ist.

7. Verfahren zum Nachweis eines Retinoblastomproteins in einer Tumorprobe, wobei die Abwesenheit des Proteins mit einem Neoplasma einhergeht und das Verfahren die Herstellung eines Antikörpers gegen das Retinoblastomprotein, das Inkontaktbringen des Antikörpers mit der Tumorprobe und den Nachweis von Immunkomplexen als Anzeichen des Vorhandenseins des Proteins in der Tumorprobe umfaßt.

8. Verfahren zum Überprüfen eines menschlichen Patienten auf das Vorhandensein eines defekten Retinoblastom (Rb)-gens, das den Vergleich der Patienten-DNA mit einem normalen menschlichen Retinoblastomgen oder einem eindeutigen Teilbereich davon umfaßt.

9. Verfahren nach Anspruch 8, wobei der Vergleich durch DNA-Hybridisierung oder durch Sequenzvergleich oder -analyse durchgeführt wird.

10. Verfahren nach Anspruch 8, wobei der Vergleich durch den Nachweis von Fehlpaarungen in einem Heteroduplex zwischen der Patienten-DNA und dem normalen Gen oder RNA-Transkripten von dem normalen Gen durchgeführt wird.

## Revendications (Revendications pour l'(les) Etat(s) contractant(s) suivant(s): BE, CH, DE, FR, GB, GR, IT, LI, LU, NL, SE)

1. Utilisation du matériel génétique correspondant au gène du rétinoblastome humain normal, ou à une sous-région unique de celui-ci, dans la préparation d'un matériel destiné à être utilisé dans un procédé de dépistage, chez des patients humains, d'allèles déficients du rétinoblastome, lequel procédé comprend la comparaison de l'ADN des patients avec le gène ou la sous-région.

2. Utilisation selon la revendication 1, dans laquelle la comparaison est effectuée par hybridation d'ADN.

3. Utilisation selon la revendication 1, dans laquelle la comparaison est effectuée par comparaison des séquences ou analyse des séquences.

4. Utilisation selon la revendication 1, dans laquelle la comparaison est effectuée par détection d'erreurs d'appariement dans un hétéroduplex entre l'ADN du patient et le gène normal ou les produits de transcription d'ARN provenant du gène normal.

5. Vecteur comprenant le matériel génétique correspondant au gène du rétinoblastome humain normal, ou à une sous-région unique de celui-ci.

6. Vecteur selon la revendication 5, dans lequel la sous-région est d'une longueur d'au moins 20 paires de bases.

7. Procédé de détection de la présence, dans un échantillon tumoral, d'une protéine du rétinoblastome dont l'absence est associée à un néoplasme, le procédé comprenant la production d'un anticorps dirigé contre la protéine du rétinoblastome, la mise en contact de l'anticorps avec l'échantillon tumoral, et la détection des complexes immuns en tant qu'indication de la présence de la protéine dans l'échantillon tumoral.

8. Procédé de dépistage chez un patient humain de la présence d'un gène déficient du rétinoblastome (Rb), le procédé comprenant la comparaison de l'ADN d'un patient avec le gène du rétinoblastome humain normal ou une sous-région unique de celui-ci.

9. Procédé selon la revendication 8, dans lequel la comparaison est effectuée par hybridation d'ADN ou par comparaison des séquences ou analyse des séquences.

10. Procédé selon la revendication 8, dans lequel la comparaison est effectuée par détection d'erreurs d'appariement dans un hétéroduplex entre l'ADN du patient et le gène normal ou les produits de transcription d'ARN provenant du gène normal.

## Revendications (Revendications pour l'(les) Etat(s) contractant(s) suivant(s): AT, ES)

1. Utilisation du matériel génétique correspondant au gène du rétinoblastome humain normal, ou à une sous-région unique de celui-ci, dans la préparation d'un matériel destiné à être utilisé dans un procédé de dépistage, chez des patients humains, d'allèles déficients du rétinoblastome, lequel procédé comprend la comparaison de l'ADN des patients avec le gène ou la sous-région.

2. Utilisation selon la revendication 1, dans laquelle la comparaison est effectuée par hybridation d'ADN.

3. Utilisation selon la revendication 1, dans laquelle la comparaison est effectuée par comparaison des séquences ou analyse des séquences.

4. Utilisation selon la revendication 1, dans laquelle la comparaison est effectuée par détection d'erreurs d'appariement dans un hétéroduplex entre l'ADN du patient et le gène normal ou les produits de transcription d'ARN provenant du gène normal.

5. Procédé de préparation d'un vecteur comprenant le matériel génétique correspondant au gène du rétinoblastome humain normal, ou à une sous-unité unique de celui-ci, le procédé comprenant ou bien la liaison de nucléotides successifs ensemble et/ou la soudure de poly- et/ou oligonucléotides.

6. Procédé selon la revendication 6, dans lequel la sous-région est d'une longueur d'au moins 20 paires de bases.

7. Procédé de détection de la présence, dans un échantillon tumoral, d'une protéine du rétinoblastome dont l'absence est associée à un néoplasme, le procédé comprenant la production d'un anticorps dirigé contre la protéine du rétinoblastome, la mise en contact de l'anticorps avec l'échantillon tumoral, et la détection des complexes immuns en tant qu'indication de la présence de la protéine dans l'échantillon tumoral.

8. Procédé de dépistage chez un patient humain de la présence d'un gène déficient du rétinoblastome (Rb), le procédé comprenant la comparaison de l'ADN d'un patient avec le gène du rétinoblastome humain normal ou une sous-région unique de celui-ci.

9. Procédé selon la revendication 8, dans lequel la comparaison est effectuée par hybridation d'ADN ou par comparaison des séquences ou analyse des séquences.

10. Procédé selon la revendication 8, dans lequel la comparaison est effectuée par détection d'erreurs d'appariement dans un hétéroduplex entre l'ADN du patient et le gène normal ou les produits de transcription d'ARN provenant du gène normal.
